# EUROPEAN PATENT SPECIFICATION

(11) **EP 3 941 286 B1**
(45) Date of publication and mention of the grant of the patent: **24.04.2024**
(21) Application number: 20715769.4
(22) Date of filing: 17.03.2020
(51) Int. Cl.: A61M 11/04, A61M 15/06, A24F 40/44, A61M 11/00, A24F 40/10, A24F 40/485

(54) **AEROSOL-GENERATION APPARATUS AND AEROSOL DELIVERY SYSTEM**
AEROSOLERZEUGUNGSSYSTEM UND AEROSOLABGABESYSTEM
DISPOSITIF DE GÉNÉRATION D'AÉROSOL ET SYSTÈME D'ADMINISTRATION D'AÉROSOL

(30) Priority: 21.03.2019 EP 19164465
(43) Date of publication of application: 26.01.2022
(73) Proprietor: Imperial Tobacco Limited, Bristol, BS3 2LL (GB)
(72) Inventor: LORD, Chris, Liverpool Merseyside L24 9HP (GB); SUDLOW, Thomas, Liverpool Merseyside L24 9HP (GB); ILLIDGE, Ben, Liverpool Merseyside L24 9HP (GB); MADDEN, Alfred, Liverpool Merseyside L24 9HP (GB); ASTBURY, Ben, Liverpool Merseyside L24 9HP (GB)
(74) Representative: Mewburn Ellis LLP
(86) International application number: PCT/EP2020/057352
(87) International publication number: WO 2020/187946

(56) References cited:
- WO-A1-2014/059790
- WO-A1-2016/154792
- WO-A1-2017/167521
- WO-A1-2018/197511
- CN-A- 107 692 328
- CN-A- 108 185 535
- CN-A- 108 514 158
- CN-U- 203 662 018
- CN-U- 205 922 888
- CN-U- 208 550 027
- US-A1- 2017 006 916
- US-A1- 2017 079 332
- US-A1- 2017 367 407
- US-A1- 2018 116 284

## Description

### Field of the Invention

The present invention relates to an aerosol delivery system and an aerosol-generation apparatus for an aerosol delivery system. In particular, the present invention relates to an aerosol delivery system including a heater configured to heat an aerosol precursor to generate an aerosolised composition for inhalation by a user, and to an aerosol-generation apparatus therefor.

### Background

Pharmaceutical medicament, physiologically active substances and flavourings for example may be delivered to the human body by inhalation through the mouth and/or nose. Such material or substances may be delivered directly to the mucosa or mucous membrane lining the nasal and oral passages and/or the pulmonary system. For example, nicotine is consumed for therapeutic or recreational purposes and may be delivered to the body in a number of ways. Nicotine replacement therapies are aimed at people who wish to stop smoking and overcome their dependence on nicotine. Nicotine is delivered to the body in the form of aerosol delivery devices and systems, also known as smoking-substitute devices or nicotine delivery devices. Such devices may be non-powered or powered.

Devices or systems that are non-powered may comprise nicotine replacement therapy devices such as "inhalators", e.g. Nicorette^{®} Inhalator. These generally have the appearance of a plastic cigarette and are used by people who crave the behaviour associated with consumption of combustible tobacco - the so-called hand-to-mouth aspect - of smoking tobacco. Inhalators generally allow nicotine-containing aerosol to be inhaled through an elongate tube in which a container containing a nicotine carrier, for example, a substrate, is located. An air stream caused by suction through the tube by the user carries nicotine vapours into the lungs of the user to satisfy a nicotine craving. The container may comprise a replaceable cartridge, which includes a cartridge housing and a passageway in the housing in which a nicotine reservoir is located. The reservoir holds a measured amount of nicotine in the form of the nicotine carrier. The measured amount of nicotine is an amount suitable for delivering a specific number of "doses". The form of the nicotine carrier is such as to allow nicotine vapour to be released into a fluid stream passing around or through the reservoir. This process is known as aerosolization and or atomization. Aerosolization is the process or act of converting a physical substance into the form of particles small and light enough to be carried on the air i.e. into an aerosol. Atomization is the process or act of separating or reducing a physical substance into fine particles and may include the generation of aerosols. The passageway generally has an opening at each end for communication with the exterior of the housing and for allowing the fluid stream through the passageway. A nicotine-impermeable barrier seals the reservoir from atmosphere. The barrier includes passageway barrier portions for sealing the passageway on both sides of the reservoir. These barrier portions are frangible so as to be penetrable for opening the passageway to atmosphere.

A device or a system that is powered can fall into two sub-categories. In both subcategories, such devices or systems may comprise electronic devices or systems that permit a user to simulate the act of smoking by producing an aerosol mist or vapour that is drawn into the lungs through the mouth and then exhaled. The electronic devices or systems typically cause the vaporization of a liquid containing nicotine and entrainment of the vapour into an airstream. Vaporization of an element or compound is a phase transition from the liquid phase to vapour i.e. evaporation or boiling. In use, the user experiences a similar satisfaction and physical sensation to those experienced from a traditional smoking or tobacco product, and exhales an aerosol mist or vapour of similar appearance to the smoke exhaled when using such traditional smoking or tobacco products.

A person of ordinary skill in the art will appreciate that devices or systems of the second, powered category as used herein include, but are not limited to, electronic nicotine delivery systems, electronic cigarettes, e-cigarettes, e-cigs, vaping cigarettes, pipes, cigars, cigarillos, vaporizers and devices of a similar nature that function to produce an aerosol mist or vapour that is inhaled by a user. Such nicotine delivery devices or systems of the second category incorporate a liquid reservoir element generally including a vaporizer or misting element such as a heating element or other suitable element, and are known, *inter alia,* as atomizers, cartomizers, or clearomizers. Some electronic cigarettes are disposable; others are reusable, with replaceable and refillable parts.

Aerosol delivery devices or systems in a first sub-category of the second, powered category generally use heat and/or ultrasonic agitation to vaporize a solution comprising nicotine and/or other flavouring, propylene glycol and/or glycerine-based base into an aerosol mist of vapour for inhalation.

Aerosol delivery devices or systems in a second sub-category of the second, powered category may typically comprise devices or systems in which tobacco is heated rather than combusted. During use, volatile compounds may be released from the tobacco by heat transfer from the heat source and entrained in air drawn through the aerosol delivery device or system. Direct contact between a heat source of the aerosol delivery device or system and the tobacco heats the tobacco to form an aerosol. As the aerosol containing the released compounds passes through the device, it cools and condenses to form an aerosol for inhalation by the user. In such devices or systems, heating, as opposed to burning, the tobacco may reduce the odour that can arise through combustion and pyrolytic degradation of tobacco.

Aerosol delivery devices or systems falling into the first sub-category of powered devices or systems may typically comprise a powered unit, comprising a heater element, which is arranged to heat a portion of a carrier that holds an aerosol precursor. The carrier comprises a substrate formed of a "wicking" material, which can absorb aerosol precursor liquid from a reservoir and hold the aerosol precursor liquid. Upon activation of the heater element, aerosol precursor liquid in the portion of the carrier in the vicinity of the heater element is vaporised and released from the carrier into an airstream flowing around the heater and carrier. Released aerosol precursor is entrained into the airstream to be borne by the airstream to an outlet of the device or system, from where it can be inhaled by a user.

The heater element is typically a resistive coil heater, which is wrapped around a portion of the carrier and is usually located in the liquid reservoir of the device or system. Consequently, the surface of the heater may always be in contact with the aerosol precursor liquid, and long-term exposure may result in the degradation of either or both of the liquid and heater. Furthermore, residues may build up upon the surface of the heater element, which may result in undesirable toxicants being inhaled by the user. Furthermore, as the level of liquid in the reservoir diminishes through use, regions of the heater element may become exposed and overheat.

The present invention has been devised in light of the above considerations.

WO 2018/197511 A1 discloses an apparatus according to the preamble of claim 1.

### Summary of the Invention

According to the invention, there is provided an aerosol-generation apparatus according to claim 1 and an aerosol delivery system according to claim 15. The dependent claims define embodiments of the invention.

At its most general, the present disclosure proposes that an aerosol-generation apparatus is provided, which has a heater and a fluid-transfer article, with the fluid-transfer article having an activation surface at an end of the article and being configured for thermal interaction with a heating surface of the heater. The activation surface has at least one channel therein, which channel opposes the heating surface and is open towards the heating surface. The heater has a substrate and at least one heating element on a part of the substrate. The fluid-transfer article is positioned so that the or each channel faces a part of the substrate other than the part of the substrate of which the or each heating element is formed.

Thus, the or each heating element is not aligned with the or each channel. Instead, it is aligned with a part of the activation surface other than that the or each channel, so that the or each heating element is aligned with the part or parts of the activation surface which project towards the heater.

Thus, the present disclosure may provide an aerosol-generation apparatus comprising a heater and a fluid-transfer article, said fluid-transfer article having a first region for holding an aerosol precursor and for transferring said aerosol precursor to an activation surface of a second region of said article, said activation surface being disposed and configured for thermal interaction with a heating surface of said heater; said second region comprising at least one discontinuity in said activation surface to form a corresponding at least one channel between said second region and said heating surface and being configured such that, when the fluid transfer article is arranged with respect to said heating surface of the heater for thermal interaction therebetween, the or each said arcuate surface portion opposes said heating surface, opens towards said heating surface and provides an air-flow pathway across said heating surface wherein the heater comprises a substrate defining said heating surface, and at least one heating element formed on a part of said heating surface, and said at least one channel opposes a further part of said heating surface other than said part of said heating surface on which said heating element is formed.

The activation surface may be disposed at an end of the fluid-transfer article.

Optionally, said activation surface is configured such that, when the fluid transfer article is arranged with respect to a said heating surface for thermal interaction therebetween, the or each said discontinuity is spaced apart from said heating surface.

Advantageously, the or each said channel is at least partly defined by a pair of spaced apart side walls, and an arcuate surface portion extending between said wall portions to form a ceiling portion of said channel.

Optionally, said arcuate surface portion blends smoothly with each of said side walls, thereby eliminating a sharp corner therebetween.

Alternatively, the or each channel may be at least partially defined by a pair of spaced apart side walls and a flat surface portion, said flat surface portion extending between said wall portions to form a ceiling portion of said channel. A further possibility is that the or each channel is at least partially defined by a pair of side walls, said side walls being inclined relative to each other to meet an apex portion of said channel.

Conveniently, said side walls are substantially planar.

Conveniently, at least said second region is formed from a polymeric wicking material.

Advantageously, said first and second regions are both formed from said polymeric wicking material.

Optionally, said polymeric wicking material is porous.

Conveniently, said polymeric wicking material is configured such that pore diameter in said first region is greater than pore diameter in said second region.

Advantageously, said polymeric wicking material is heat resistant.

Optionally, said polymeric wicking material is a hydrophilic material that is configured to transfer fluid from said first region to said second region.

Conveniently, said polymeric wicking material is of greater hydrophilicity in said second region than said first region.

According to another aspect of the present disclosure, there may be provided an aerosol delivery system comprising an aerosol-generation apparatus as discussed above, and a carrier, which carrier has a housing containing the heater and the fluid-transfer article.

Preferably, the housing has an inlet and an outlet, with the air-flow pathway extending between the inlet and outlet.

The invention includes the combination of the aspects and preferred features described except where such a combination is clearly impermissible or expressly avoided, provided the resulting combination of features falls within the scope of the claims.

### Summary of the Figures

So that the invention may be more readily understood, and so that further features thereof may be appreciated, embodiments of the invention will now be described by way of example with reference to the accompanying drawings in which:
**Figure 1****.** is a perspective view illustration of a system for aerosol delivery according to one or more embodiments of the present invention;
**Figure 2****.** is a cross-sectional side view illustration of part of an apparatus of the system for aerosol delivery of Figure 1;
**Figure 3****.** is a cross-sectional side view illustration of the system and apparatus for aerosol delivery of Figure 1;
**Figure 4****.** is a perspective view illustration of an aerosol carrier for use in the system for aerosol delivery according to one or more embodiments of the present invention;
**Figure 5****.** is a cross-section side view of elements of an aerosol carrier and of part of an apparatus of the system for aerosol delivery according to one or more embodiments of the present invention;
**Figure 6****.** is a cross-section side view of elements of an aerosol carrier and of part of an apparatus of the system for aerosol delivery according to one or more embodiments of the present invention;
**Figure 7****.** is a perspective view illustration of the aerosol carrier and of part of an apparatus of the system for aerosol delivery according to one or more embodiments of the present invention;
**Figure 8****.** is a perspective view illustration of the aerosol carrier and of part of an apparatus of the system for aerosol delivery according to one or more embodiments of the present invention;
**Figure 9****.** is a perspective end view illustration of a fluid-transfer article of the aerosol carrier according to one or more embodiments of the present invention;
**Figure 10****.** is a perspective end view illustration of a fluid-transfer article of the aerosol carried according to one or more embodiments of the present invention;
**Figure 11****.** is a cross-section side view of an aerosol carrier according to one or more embodiments of the present invention;
**Figure 12****.** is a perspective cross-section side view of the aerosol carrier of Figure 11;
**Figure 13****.** is an exploded perspective view illustration of a kit-of-parts for assembling a system according to one or more embodiments of the present invention;
**Figure 14****.** is a cross-section side view of elements of an aerosol carrier and of part of an apparatus of the system for aerosol delivery according to one or more embodiments of the present invention; and
**Figure 15****.** is a perspective view of elements of an aerosol carrier and of part of an apparatus of the system for aerosol delivery according to one or more embodiments of the present invention;

### Detailed Description of the Invention

Aspects and embodiments of the present invention will now be discussed with reference to the accompanying figures. Further aspects and embodiments will be apparent to those skilled in the art.

In general outline, one or more embodiments in accordance with the present invention may provide a system for aerosol delivery in which an aerosol carrier may be inserted into a receptacle (e.g. a "heating chamber") of an apparatus for initiating and maintaining release of an aerosol from the aerosol carrier. Another end, or another end portion, of the aerosol carrier may protrude from the apparatus and can be inserted into the mouth of a user for the inhalation of aerosol released from the aerosol carrier cartridge during operation of the apparatus.

Hereinafter, and for convenience only, "system for aerosol delivery" shall be referred to as "aerosol delivery system".

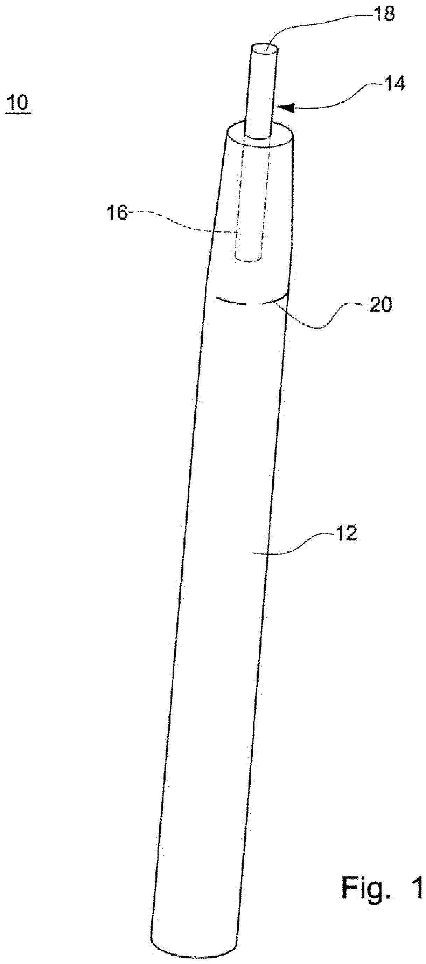

Referring now to Figure 1, there is illustrated a perspective view of an aerosol delivery system 10 comprising an aerosol generation apparatus 12 operative to initiate and maintain release of aerosol from a fluid-transfer article in an aerosol carrier 14. In the arrangement of Figure 1, the aerosol carrier 14 is shown with a first end 16 thereof and a portion of the length of the aerosol carrier 14 located within a receptacle of the apparatus 12. A remaining portion of the aerosol carrier 14 extends out of the receptacle. This remaining portion of the aerosol carrier 14, terminating at a second end 18 of the aerosol carrier, is configured for insertion into a user's mouth. A vapour and/or aerosol is produced when a heater (not shown in Figure 1) of the apparatus 12 heats a fluid-transfer article in the aerosol carrier 14 to release a vapour and/or an aerosol, and this can be delivered to the user, when the user sucks or inhales, via a fluid passage in communication with an outlet of the aerosol carrier 14 from the fluid-transfer article to the second end 18.

The device 12 also comprises air-intake apertures 20 in the housing of the apparatus 12 to provide a passage for air to be drawn into the interior of the apparatus 12 (when the user sucks or inhales) for delivery to the first end 16 of the aerosol carrier 14, so that the air can be drawn across an activation surface of a fluid-transfer article located within a housing of the aerosol carrier cartridge 14 during use. Optionally, these apertures may be perforations in the housing of the apparatus 12.

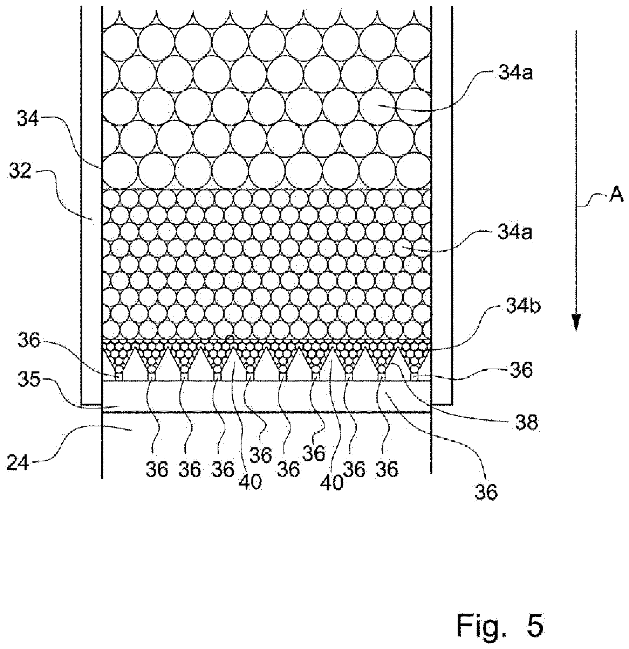

A fluid-transfer article (not shown in Figure 1, but described hereinafter with reference to Figs. 5, 6, 7, 8, 9, 10, 11 and 12) is located within a housing of the aerosol carrier 14. The fluid-transfer article contains an aerosol precursor material, which may include at least one of: nicotine; a nicotine precursor material; a nicotine compound; and one or more flavourings. The fluid-transfer article is located within the housing of the aerosol carrier 14 to allow air drawn into the aerosol carrier 14 at, or proximal, the first end 16 to flow across an activation surface of the fluid-transfer article. As air passes across the activation surface of the fluid-transfer article, an aerosol may be entrained in the air stream from a substrate forming the fluid-transfer article, e.g. via diffusion from the substrate to the air stream and/or via vaporisation of the aerosol precursor material and release from the fluid-transfer article under heating.

The substrate forming the fluid-transfer article 34 comprises a porous material where pores of the porous material hold, contain, carry, or bear the aerosol precursor material. In particular, the porous material of the fluid-transfer article may be a polymeric wicking material such as, for example, a sintered material. Particular examples of material suitable for the fluid-transfer article include: Polyetherimide (PEI); Polytetrafluoroethylene (PTFE); Polyether ether ketone (PEEK); Polyimide (PI); Polyethersulphone (PES); and Ultra-High Molecular Weight Polyethylene. Other suitable materials may comprise, for example, BioVyonTM (by Porvair Filtration Group Ltd) and materials available from Porex^{®}. Further optionally, a substrate forming the fluid-transfer article may comprise Polypropylene (PP) or Polyethylene Terephthalate (PET). All such materials may be described as heat resistant polymeric wicking material in the context of the present invention.

The aerosol carrier 14 is removable from the apparatus 12 so that it may be disposed of when expired. After removal of a used aerosol carrier 14, a replacement aerosol carrier 14 can be inserted into the apparatus 12 to replace the used aerosol carrier 14.

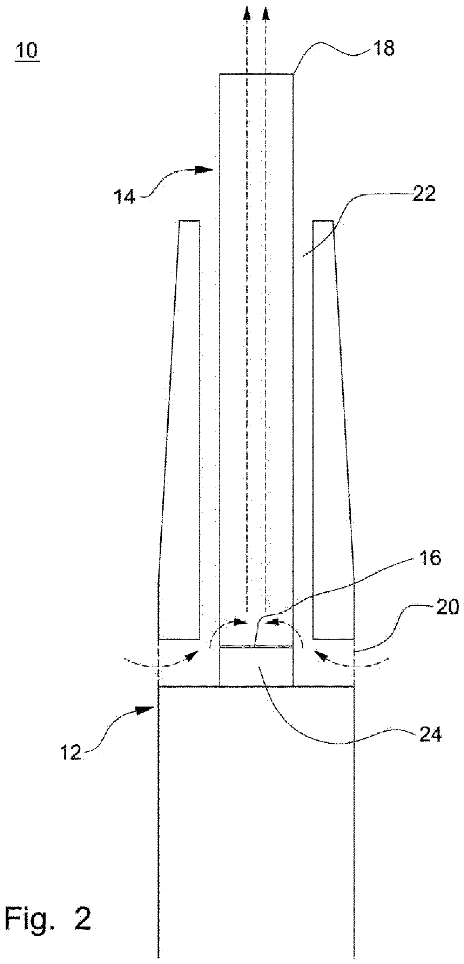

Figure 2 is a cross-sectional side view illustration of a part of apparatus 12 of the aerosol delivery system 10. The apparatus 12 comprises a receptacle 22 in which is located a portion of the aerosol carrier 14. In one or more optional arrangements, the receptacle 22 may enclose the aerosol carrier 14. The apparatus 12 also comprise a heater 24, which opposes an activation surface of the fluid-transfer article (not shown in Figure 2) of the aerosol carrier 14 when an aerosol carrier 14 is located within the receptacle 22.

Air flows into the apparatus 12 (in particular, into a closed end of the receptacle 22) via air-intake apertures 20. From the closed end of the receptacle 22, the air is drawn into the aerosol carrier 14 (under the action of the user inhaling or sucking on the second end 18) and expelled at the second end 18. As the air flows into the aerosol carrier 14, it passes across the activation surface of the fluid-transfer article. Heat from the heater 24, which opposes the activation surface of the fluid-transfer article, causes vaporisation of aerosol precursor material at the activation surface of the fluid-transfer article and an aerosol is created in the air flowing over the activation surface. Thus, through the application of heat in the region of the activation surface of the fluid-transfer article, an aerosol is released, or liberated, from the fluid-transfer article, and is drawn from the material of the aerosol carrier unit by the air flowing across the activation surface and is transported in the air flow to via outlet conduits (not shown in Figure 2) in the housing of the aerosol carrier 14 to the second end 18. The direction of air flow is illustrated by arrows in Figure 2.

To achieve release of the captive aerosol from the fluid-transfer article, the fluid-transfer article of the aerosol carrier 14 is heated by the heater 24. As a user sucks or inhales on second end 18 of the aerosol carrier 14, the aerosol released from the fluid-transfer article and entrained in the air flowing across the activation surface of the fluid-transfer article is drawn through the outlet conduits (not shown) in the housing of the aerosol carrier 14 towards the second end 18 and onwards into the user's mouth.

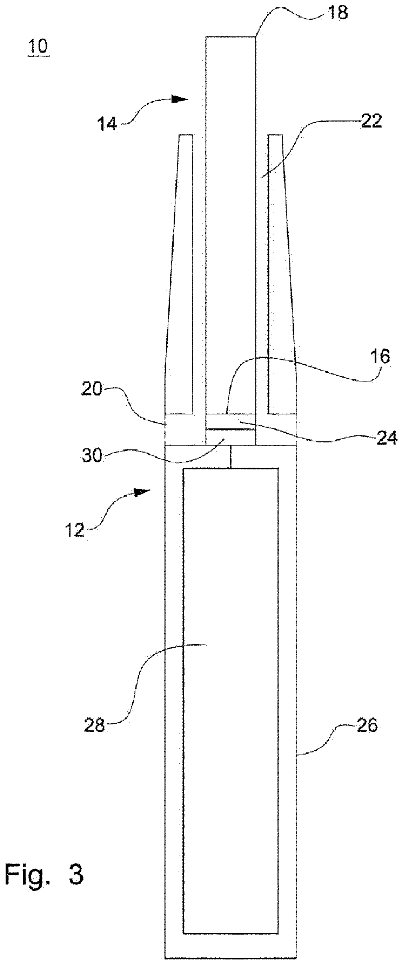

Turning now to Figure 3, a cross-sectional side view of the aerosol delivery system 10 is schematically illustrated showing the features described above in relation to Figures 1 and 2 in more detail.

As can be seen, apparatus 12 comprises a housing 26, in which are located the receptacle 22 and heater 24. The housing 26 also contains control circuitry (not shown) operative by a user, or upon detection of air and/or vapour being drawn into the device 12 through air-intake apertures 20, i.e. when the user sucks or inhales. Additionally, the housing 26 comprises an electrical energy supply 28, for example a battery. Optionally, the battery comprises a rechargeable lithium ion battery. The housing 26 also comprises a coupling 30 for electrically (and optionally mechanically) coupling the electrical energy supply 28 to control circuitry (not shown) for powering and controlling operation of the heater 24.

Responsive to activation of the control circuitry of apparatus 12, the heater 24 heats the fluid-transfer article (not shown in Figure 3) of aerosol carrier 14. This heating process initiates (and, through continued operation, maintains) release of vapour and/or an aerosol from the activation surface of the fluid-transfer article. The vapour and/or aerosol formed as a result of the heating process is entrained into a stream of air being drawn across the activation surface of the fluid-transfer article (as the user sucks or inhales). The stream of air with the entrained vapour and/or aerosol passes through the aerosol carrier 14 via outlet conduits (not shown) and exits the aerosol carrier 14 at second end 18 for delivery to the user.

This process is briefly described above in relation to Figure 2, where arrows schematically denote the flow of the air stream into the device 12 and through the aerosol carrier 14, and the flow of the air stream with the entrained vapour and/or aerosol through the aerosol carrier cartridge 14.

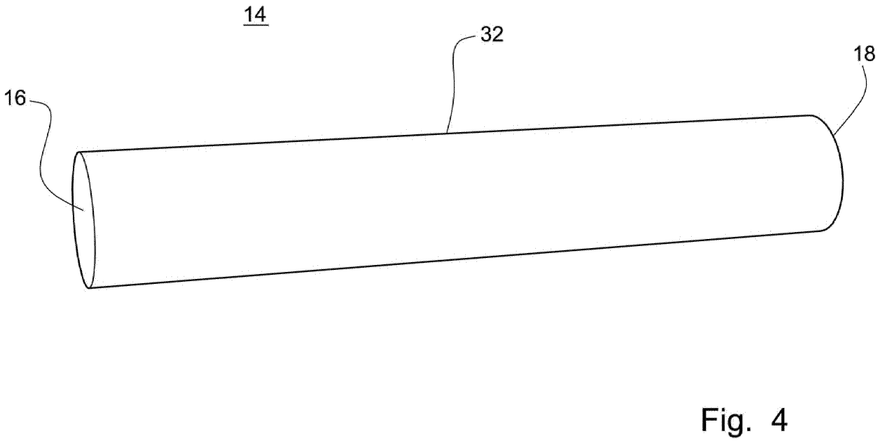

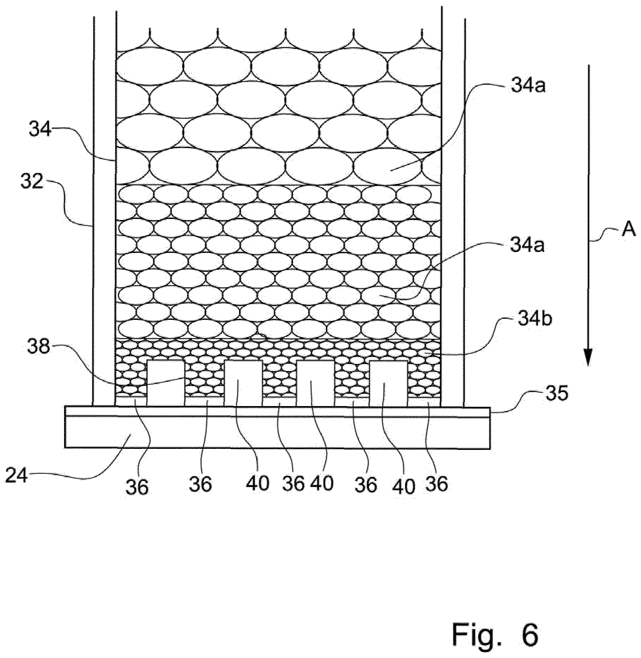

Figures 4 to 6 schematically illustrate the aerosol carrier 14 in more detail (and, in Figures 5 and 6, features within the receptacle in more detail). Figure 4 illustrates an exterior of the aerosol carrier 14, Figure 5 illustrates internal components of the aerosol carrier 14 in an optional arrangement, and Fig. 6 illustrates internal components of the aerosol carrier 14 in another optional arrangement.

Fig. 4 illustrates the exterior of the aerosol carrier 14, which comprises housing 32 for housing said fluid-transfer article (not shown) and at least one other internal component. The particular housing 32 illustrated in Figure 4 comprises a tubular member, which may be generally cylindrical in form, and which is configured to be received within the receptacle of the apparatus. First end 16 of the aerosol carrier 14 is for location to oppose the heater of the apparatus, and second end 18 (and the region adjacent the second end 18) is configured for insertion into a user's mouth.

Figure 5 illustrates some internal components of the aerosol carrier 14 and of the heater 24 of apparatus 12.

As described above, the aerosol carrier 14 comprises a fluid-transfer article 34. The aerosol carrier 14 optionally may comprise a conduction element 36 (as shown in Figure 5). In one or more arrangements, the aerosol carrier 14 is located within the receptacle of the apparatus such that the activation surface of the fluid-transfer article opposes the heater of the apparatus and receives heat directly from the heater of the apparatus. In an optional arrangement, such as illustrated in Figure 5 for example, the aerosol carrier 14 comprises a conduction element 36. When aerosol carrier 14 is located within the receptacle of the apparatus such that the activation surface of the fluid-transfer article is located to oppose the heater of the apparatus, the conduction element is disposed between the heater 24 and the activation surface of the fluid-transfer article. Heat may be transferred to the activation surface via conduction through conduction element 36 (i.e. application of heat to the activation surface is indirect).

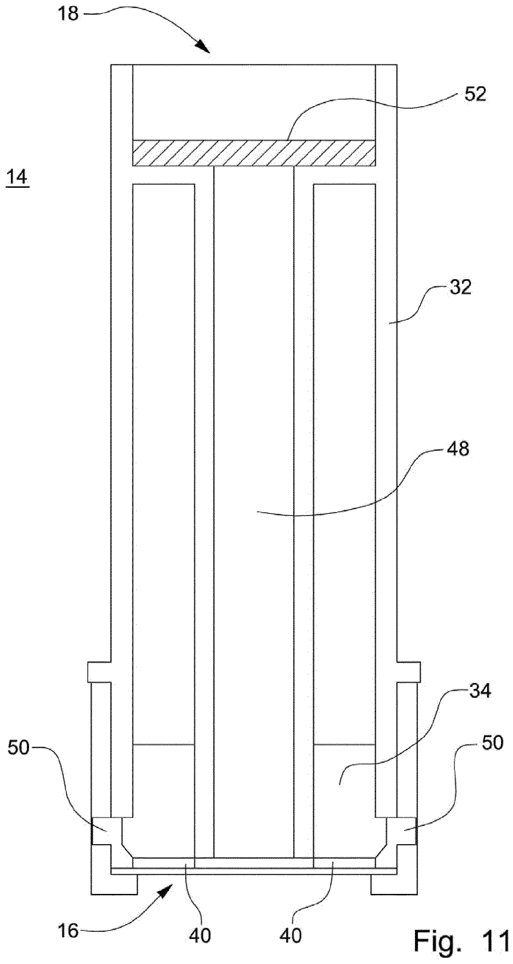

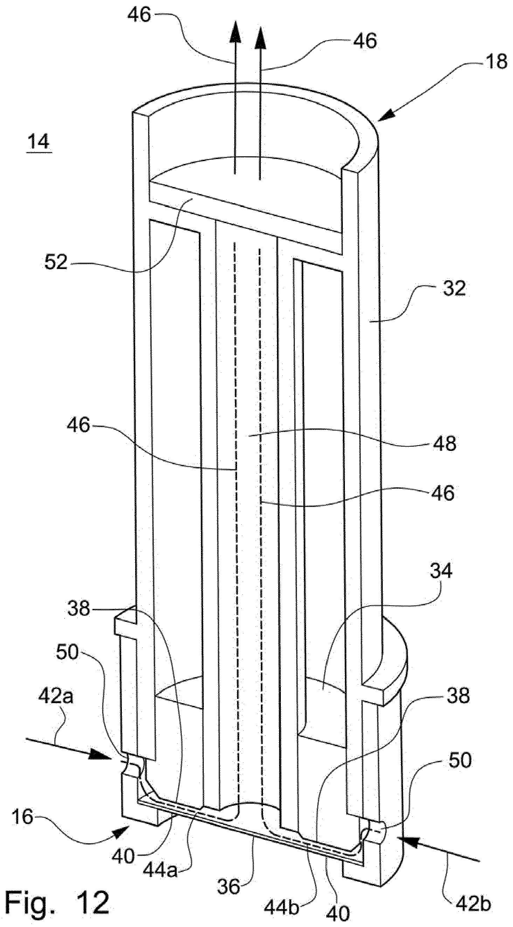

Further components not shown in Figures. 5 and 6 (see Figures 11 and 12) comprise: an inlet conduit, via which air can be drawn into the aerosol carrier 14; an outlet conduit, via which an air stream entrained with aerosol can be drawn from the aerosol carrier 14; a filter element; and a reservoir for storing aerosol precursor material and for providing the aerosol precursor material to the fluid-transfer article 34.

In Figures 5 and 6, aerosol carrier is shown as comprising the fluid-transfer article 34 located within housing 32. The material forming the fluid transfer article 34 comprises a porous structure, where pore diameter size varies between one end of the fluid-transfer article 34 and another end of the fluid-transfer article. In the illustrative examples of Figs. 5 and 6, the pore diameter size gradually decreases from a first end remote from heater 24 (the upper end as shown in the figure) to a second end proximal heater 24 (the lower end as shown in the figure). Although the figure illustrates the pore diameter size changing in a step-wise manner from the first to the second end (i.e. a first region with pores having a diameter of a first size, a second region with pores having a diameter of a second, smaller size, and a third region with pores having a diameter of a third, yet smaller size), the change in pore size from the first end to the second end may be gradual rather than step-wise. This configuration of pores having a decreasing diameter size from the first end and second end can provide a wicking effect, which can serve to draw fluid from the first end to the second end of the fluid-transfer article 34.

The fluid-transfer article 34 comprises a first region 34a for holding an aerosol precursor. In one or more arrangements, the first region 34a of the fluid-transfer article 34 comprises a reservoir for holding the aerosol precursor. The first region 34a can be the sole reservoir of the aerosol carrier 14, or it can be arranged in fluid communication with a separate reservoir, where aerosol precursor is stored for supply to the first region 34a.

The fluid-transfer article 34 also comprises a second region 34b. Aerosol precursor is drawn from the first region 34a to the second region 34b by the wicking effect of the substrate material forming the fluid transfer article. Thus, the first region 34a is configured to transfer the aerosol precursor to the second region 34b of the article 34.

At the second end of fluid-transfer article 34, the surface of the second region 34b defines an activation surface 38, which is disposed opposite a surface for conveying heat to the activation surface 38. In the illustrative examples of Figures. 5 and 6, the opposing surface for conveying heat to the activation surface 38 comprises part of the heater 24 being a substrate 35 which has heating elements 36 thereon. The elements 36 will be powered individually, or may be connected together so that they are powered together. The heating elements 36 generate heat, when they are activated. Thus, those heating elements are located for thermal interaction with the second region 34b, and arranged to transfer heat from the activation surface 38.

The activation surface 38 is discontinuous such that at least one channel 40 is formed between the activation surface 38 and the heater 24. In some arrangements, the discontinuities may be such that the activation surface 38 is undulating.

In the illustrative examples of Figures 5 and 6, the activation surface 38 comprises a plurality of grooves or valleys therein to form an undulating surface, the grooves or valleys being disposed in a parallel arrangement across the activation surface 38. Thus, there are a plurality of channels 40 between the activation surface 38 and the heater 24.

In the illustrative example of Figure 5, the grooves or valleys in the activation surface 38 provide alternating peaks and troughs that give rise to a "saw-tooth" type profile. In one or more optional arrangements, the activation surface may comprise a "castellated" type profile (i.e. a "square wave" type profile), for example, such as illustrated in the example of Figure. 6. In one or more optional arrangements, the activation surface may comprise a "sinusoidal" type profile. The profile may comprise a mixture of two or more of the above profiles given as illustrative examples.

As can be seen in Figures 5 and 6 the heating elements are not aligned with the channels 40, but instead are aligned with the parts of the activation surface between the grooves or valleys, i.e. the parts of the second region 34b which are closest to the heater 24. There may be direct contact between those parts of the second region 34b and the heating elements 36. The heating elements 36 thus heat the activation surface 38 at the walls between the troughs or valleys, rather than being aligned with the troughs or valleys themselves. Heat may then reach the rest of the activation surface 38 by conduction through the second region 34b and also by radiation across the channels 40.

In the illustrative examples of Figures 5 and 6, the first region 34a of the fluid-transfer article 34 is located at an "upstream" end of the fluid-transfer article 34 and the second region 34b is located at a downstream" end of the fluid-transfer article 34. That is, aerosol precursor is wicked, or is drawn, from the "upstream" end of the fluid-transfer article 34 to the "downstream" end of the fluid-transfer article 34 (as denoted by arrow A in Figure 5).

The aerosol precursor is configured to release an aerosol and/or vapour upon heating. Thus, when the activation surface 38 receives heat conveyed from heater 24, the aerosol precursor held at the activation surface 38 is heated. The aerosol precursor, which is captively held in material of the fluid-transfer article at the activation surface 38 is released into an air stream flowing through the channels 40 between the heater 24 and activation surface 38 as an aerosol and/or vapour.

The shape and/or configuration of the activation surface 38 and the associated shape(s) and/or configuration(s) of the one or more channels 40 formed between the activation surface 38 and heater 24 permit air to flow across the activation surface 38 (through the one or more channels 40) and also increase the surface area of the activation surface 38 of the fluid-transfer article 34 that is available for contact with a flow of air across the activation surface 38.

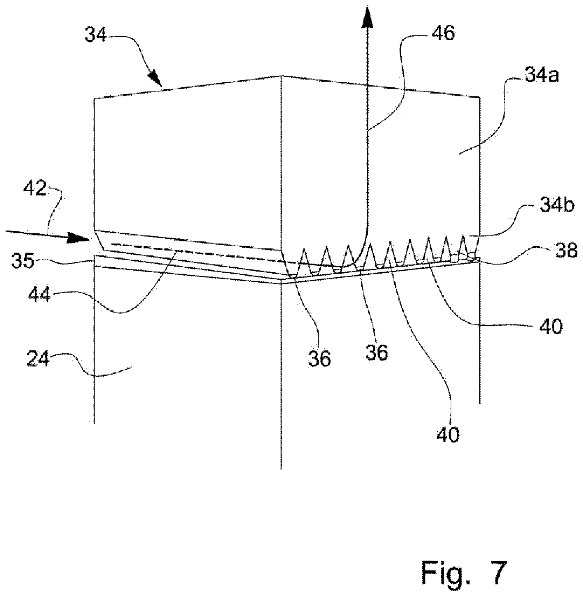

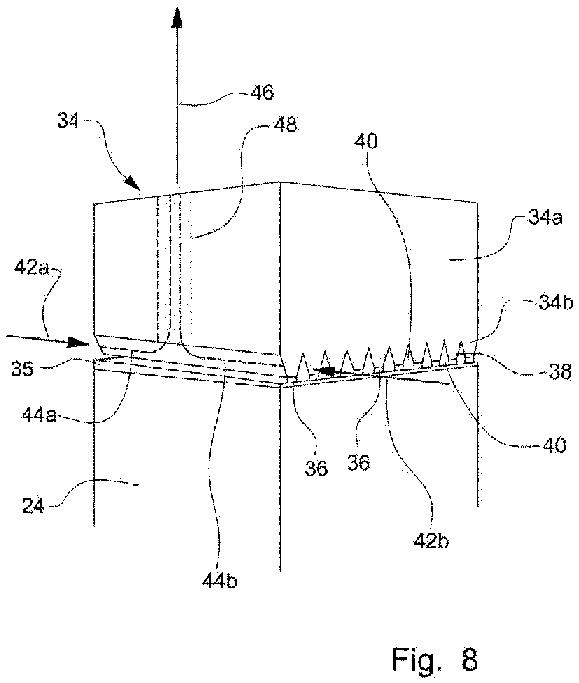

Figures 7 and 8 show perspective view illustrations of the fluid-transfer article 34 of aerosol carrier and a heater 24 of the apparatus of the system for aerosol delivery. In particular, these figures illustrate air flows across the activation surface 38 when the apparatus is in use in a first arrangement of the fluid-transfer article 34 (see Figure 7), and in a second arrangement of the fluid-transfer article 34 (see Figure 8).

In the illustrated example of use of the apparatus schematically illustrated in Figure 7, when a user sucks on a mouthpiece of the apparatus, air is drawn into the carrier through inlet apertures (not shown) provided in a housing of the carrier. An incoming air stream 42 is directed to the activation surface 38 of the fluid-transfer article 34 (e.g. via a fluid communication pathway within the housing of the carrier). When the incoming air stream 42 reaches a first side of the activation surface 38, the incoming air stream 42 flows across the activation surface 38 via the one or more channels 40 formed between the activation surface 38 and heater 24. The air stream flowing through the one or more channels 40 is denoted by dashed line 44 in Figure 7. As the air stream 44 flows through the one or more channels 40, aerosol precursor at activation surface 38, across which the air stream 44 flows, is released from the activation surface 38 by heat conveyed to the activation surface from the heater 24. Aerosol precursor released from the activation surface 38 in this manner is then entrained in the air stream 44 flowing through the one or more channels 40.

In use, the heater 24 of the apparatus 12 conveys heat to the fluid transfer article 34 to raise the temperature of the activation surface 38 to a sufficient temperature to release, or liberate, captive substances (i.e. the aerosol precursor) held at the activation surface 38 of the fluid-transfer article 34 to form a vapour and/or aerosol, which is drawn downstream across the activation surface 38 of the fluid-transfer article. As the air stream 44 continues its passage in the one or more channels 40, more released aerosol precursor is entrained within the air stream 44. When the air stream 44 entrained with aerosol precursor exits the one or more channels 40 at a second side of the activation surface 38, it is directed to an outlet, from where it can be inhaled by the user via a mouthpiece. An outgoing air stream 46 entrained with aerosol precursor is directed to the outlet (e.g. via a fluid communication pathway within the housing of the carrier).

Therefore, operation of the apparatus will cause heat from the heater 24 to be conveyed to the activation surface 38 of the fluid-transfer article. At a sufficiently high temperature, captive substances held at the activation surface 38 of the fluid-transfer article 34 are released, or liberated, to form a vapour and/or aerosol. Thus, when a user draws on a mouthpiece of the apparatus, the released substances from the fluid-transfer article are drawn away from the activation surface 38 (entrained in a stream of air) and condense to form an aerosol that is drawn through the a gas communication pathway for delivery to an outlet, which is in fluid communication with the mouthpiece.

As the aerosol precursor is released from the activation surface 38, a wicking effect of the fluid-transfer article 34 causes aerosol precursor within the body of the fluid-transfer article to migrate to the activation surface 38 to replace the aerosol precursor released from the activation surface 38 into air stream 44.

Operation of the heater 24 is controlled by control circuitry (not shown), which is operable to actuate the heater 24 responsive to an actuation signal from a switch operable by a user or configured to detect when the user draws air through a mouthpiece of the apparatus by sucking or inhaling. In an optional arrangement, the control circuitry operates to actuate the heater 24 with as little delay as possible from receipt of the actuation signal from the switch, or detection of the user drawing air through the mouthpiece. This may effect near instantaneous heating of the activation surface 38 of the fluid-transfer article 34.

In the illustrated example of use of the apparatus schematically illustrated in Figure 8, rather than the case of Figure 7, where air is drawn toward the activation surface 38 from one side only (and exits from the one or more channels 40 at an opposite side), a gas communication pathway for an incoming air stream is configured to deliver the incoming air stream to the activation surface 38 from both sides of the fluid-transfer article, and thus from both ends of the channels 40 formed therein. In such an arrangement, a gas communication pathway for an outlet airstream may be provided through the body of the fluid-transfer article 34. An outlet fluid communication pathway for an outlet airstream in the illustrative example of Figure 8 is denoted by reference number 48.

Thus, in the illustrative example of Figure 8, when a user draws on a mouthpiece of the apparatus, air is drawn into the carrier 14 through inlet apertures (not shown) provided in a housing of the carrier. An incoming air stream 42a from a first side is directed to a first side of the activation surface 38 of the fluid-transfer article 34 (e.g. via a gas communication pathway within the housing of the carrier 14). An incoming air stream 42b from a second side is directed to a second side of the activation surface 38 of the fluid-transfer article 34 (e.g. via a gas communication pathway within the housing of the carrier 14). When the incoming air stream 42a from the first side reaches the first side of the activation surface 38, the incoming air stream 42a flows across the activation surface 38 via the one or more channels 40 formed between the activation surface 38 and the heater 24. Likewise, when the incoming air stream 42b from the second side reaches the second side of the activation surface 38, the incoming air stream 42b flows across the activation surface 38 via the one or more channels 40 formed between the activation surface 38 and the heater 24. The air streams 42a, 42b from each side flowing through the one or more channels 40 are denoted by dashed lines 44a and 44b in Figure 8. As air streams 44a and 44b flow through the one or more channels 40, aerosol precursor in the activation surface 38, across which the air streams 44a and 44b flow, is released from the activation surface 38 by heat conveyed to the activation surface from the heater 24. Aerosol precursor released from the activation surface 38 is entrained in air streams 44a and 44b flowing through the one or more channels 40.

In use, the heater 24 of the apparatus 12 conveys heat to the fluid-transfer article 34 to raise a temperature of the activation surface 38 to a sufficient temperature to release, or liberate, captive substances (i.e. the aerosol precursor) held at the activation surface 38 of the fluid-transfer article 34 to form a vapour and/or aerosol, which is drawn downstream across the activation surface 38 of the fluid-transfer article. As the air streams 44a and 44b continue their passages in the one or more channels 40, more released aerosol precursor is entrained within the air streams 44a and 44b. When the air streams 44a and 44b entrained with aerosol precursor meet at a mouth of the outlet fluid communication pathway 48, they enter the outlet fluid communication pathway 48 and continue until they exit outlet fluid communication pathway 48, either as a single outgoing air stream 46 (as shown), or as separate outgoing air streams. The outgoing air stream 46 is directed to an outlet, from where it can be inhaled by the user via a mouthpiece. The outgoing air stream 46 entrained with aerosol precursor is directed to the outlet (e.g. via a gas communication pathway within the housing of the carrier 14).

It should be noted that, in Figures 7 and 8, heater 24 similar to that in Figures 5 and 6, with a substrate 35 on which are formed heating elements 36. Those heating elements are not aligned with the channels 40, but are aligned with the walls between those channels 40.

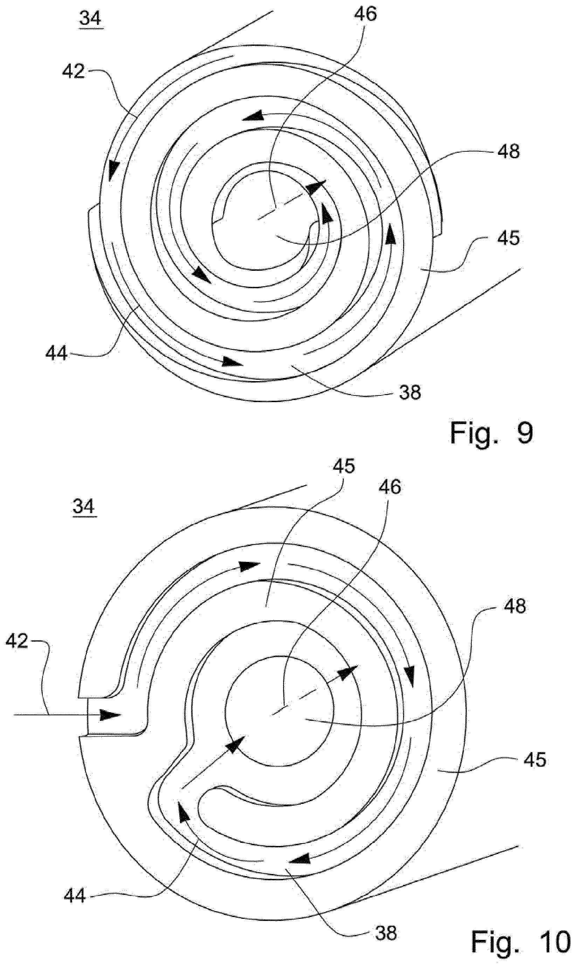

Figures 9 and 10 are perspective end view illustrations of a fluid-transfer article 34 of the aerosol carrier according to one or more arrangements. These figures show different types of channel configurations as illustrative examples. In both illustrative examples of a channel configuration, as shown in Figures 9 and 10, the fluid-transfer article 34 comprises a cylindrical member, which comprises a central bore extending therethrough for fluid communication between the activation surface 38 and an outlet, from where an outgoing air stream can be delivered for inhalation. The central bore serves as a fluid communication pathway 48 (e.g. as described above in relation to Figure 9). Note that, in the arrangements of Figures 9 and 10, the channels 40 extend radially and the sectional views of Figures 9 and 10 are along the length of two channels on opposite radial positions relative to the central bore of the fluid-transfer article. The heating elements 36 are therefore not visible in Figures 9 and 10, although they will be in similar positions, relative to the channels, as the heating elements 36 and channels in Figures 5 to 8.

In both illustrative examples of Figures 9 and 10, an incoming air stream 42 is directed to a mouth of a channel 40 formed between the activation surface 38 of the fluid-transfer article 34 and conduction element (not shown), or between the activation surface 38 and a heater (not shown). In both illustrative examples of Figures 9 and 10, the mouth of the channel 40 is located at an outer edge of the fluid-transfer article 34 and an exit from the channel 40 (in fluid communication with the fluid communication pathway 48) is located toward a centre of the fluid-transfer article. Therefore, the incoming air stream 42 enters the channel 40 via channel mouth at the outer edge of the fluid-transfer article 34 and moves toward the centre of the fluid-transfer article 34 as directed by the channel 40. As described above, as the air stream passes across activation surface 38 through channel 40, aerosol precursor is released from the activation surface 38 and is entrained in air stream 44. Air stream 44 continues to flow through the channel 40 until it reaches an exit thereof, from where it enters the fluid communication pathway 48 and proceeds as an outgoing air stream 46 entrained with aerosol precursor toward the outlet.

In both illustrative examples of Figures 9 and 10, the valleys or grooves of the activation surface 38 that form part of the channel 40 are arranged to define a circuitous route 20 across the activation surface. In the illustrative examples, the route is a spiral path, but in optional arrangements, may be meandering or circuitous in some other manner. In optional arrangements, the activation surface may be located to face outwardly from the cylinder, such that the groove(s) or valley(s) may be in the outer surface of the cylinder forming the fluid-transfer article. These grooves or valleys may be arranged in parallel in a direction along the length of the cylinder. The groove(s) or valley(s) may be arranged in a spiral manner around the outside of the cylinder. In optional arrangements, the activation surface 38 may be located to face inwardly from the cylinder (i.e. surrounding the central bore), such that the groove(s) or valley(s) may be in the inner surface of the cylinder forming the fluid-transfer article 34. These grooves or valleys may be arranged in parallel in a direction along the length of the cylinder. The groove(s) or valley(s) may be arranged in a spiral manner around the inside of the cylinder.

With the arrangement shown in Figures 9 and 10, the heating elements of the heater are therein not aligned with the valleys or grooves in the activation surface 38. Instead, they will be aligned with the projecting wall 45 of the activation surface 38, between which walls 45 the valleys or grooves are formed.

Figures 11 and 12 illustrate an aerosol carrier 14 according to one or more possible arrangements in more detail. Figure 11 is a cross-section side view illustration of the aerosol carrier 14 and Figure 12 is a perspective cross-section side view illustration of the aerosol carrier 14 of Figure 11. In Figures 11 and 12, the structure of the heater 24 is not illustrated in detail. However, it may correspond to e.g. one of the arrangements of Figures 5 to 8, with a heater 24 having a substrate 35 on which heating elements 36 are formed which are not aligned with the channels 40, and instead are aligned with the parts of the activation surface between those channels 40.

As can be seen from Figures 11 and 12, the aerosol carrier 14 is generally tubular in form. The aerosol carrier 14 comprises housing 32, which defines the external walls of the aerosol carrier 14 and which defines therein a chamber in which are disposed the fluid-transfer article 34 (adjacent the first end 16 of the aerosol carrier 14) and internal walls defining the fluid communication pathway 48. Fluid communication pathway 48 defines a fluid pathway for an outgoing air stream from the channels 40 to the second end 18 of the aerosol carrier 14. In the examples illustrated in Figures 11 and 12, the fluid-transfer article 34 is an annular shaped element located around the fluid communication pathway 48, and the channels 40 are arranged so as to extend radially across its activation surface.

In walls of the housing 32, there are provided inlet apertures 50 to provide a fluid communication pathway for an incoming air stream to reach the fluid-transfer article 34, and particularly the one or more channels 40 defined between the activation surface of the fluid-transfer article 34 and the heater 24.

In the illustrated example of Figures 11 and 12, the aerosol carrier 14 further comprises a filter element 52. The filter element 52 is located across the fluid communication pathway 48 such that an outgoing air stream passing through the fluid communication pathway 48 passes through the filter element 52.

With reference to Figure 12, when a user sucks on a mouthpiece of the apparatus (or on the second end 18 of the aerosol carrier 14, if configured as a mouthpiece), air is drawn into the carrier through inlet apertures 50 extending through walls in the housing 32 of the aerosol carrier 14. An incoming air stream 42a from a first side of the aerosol carrier 14 is directed to a first side of the activation surface 38 of the fluid-transfer article 34 (e.g. via a gas communication pathway within the housing of the carrier). An incoming air stream 42b from a second side of the aerosol carrier 14 is directed to a second side of the activation surface 38 of the fluid-transfer article 34 (e.g. via a gas communication pathway within the housing of the carrier). When the incoming air stream 42a from the first side of the aerosol carrier 14 reaches the first side of the activation surface 38, the incoming air stream 42a from the first side of the aerosol carrier 14 flows across the activation surface 38 via the one or more channels 40 formed between the activation surface 38 and the conduction element 36 (or between the activation surface 38 and heater 24). Likewise, when the incoming air stream 42b from the second side of the aerosol carrier 14 reaches the second side of the activation surface 38, the incoming air stream 42b from the second side of the aerosol carrier 14 flows across the activation surface 38 via the one or more channels 40 formed between the activation surface 38 and the conduction element 36 (or between the activation surface 38 and heater 24). The air streams from each side flowing through the one or more channels 40 are denoted by dashed lines 44a and 44b in Figure 12. As air streams 44a and 44b flow through the one or more channels 40, aerosol precursor in the activation surface 38, across which the air streams 44a and 44b flow, is released from the activation surface 38 by heat conveyed to the activation surface from the heater 24. Aerosol precursor released from the activation surface 38 is entrained in air streams 44a and 44b flowing through the one or more channels 40.

In use, the heater 24 of the apparatus 12 conveys heat to the activation surface 38 of the fluid-transfer article 34 to raise a temperature of the activation surface 38 to a sufficient temperature to release, or liberate, captive substances (i.e. the aerosol precursor) held at the activation surface 38 of the fluid-transfer article 34 to form a vapour and/or aerosol, which is drawn downstream across the activation surface 38 of the fluid-transfer article 34. As the air streams 44a and 44b continue their passages in the one or more channels 40, more released aerosol precursor is entrained within the air streams 44a and 44b. When the air streams 44a and 44b entrained with aerosol precursor meet at a mouth of the outlet fluid communication pathway 48, they enter the outlet fluid communication pathway 48 and continue until they pass through filter element 52 and exit outlet fluid communication pathway 48, either as a single outgoing air stream, or as separate outgoing air streams 46 (as shown). The outgoing air streams 46 are directed to an outlet, from where it can be inhaled by the user directly (if the second end 18 of the aerosol capsule 14 is configured as a mouthpiece), or via a mouthpiece. The outgoing air streams 46 entrained with aerosol precursor are directed to the outlet (e.g. via a gas communication pathway within the housing of the carrier).

When the user initially draws on a mouthpiece of the apparatus (or one the second end 18 of the aerosol carrier 14, if configured as a mouthpiece), this will cause an air column located in the fluid communication pathway 48 to move towards the outlet. In turn, this will draw air into the fluid communication pathway from the one or more channels 40. This will cause a pressure drop in the channels 40. To equalise the pressure in the channels 40, air will be drawn into the aerosol carrier 14, and thus into the channels 40 via the inlet apertures 50. During the period of lower pressure in the one or more channels 40 when the user begins to draw, aerosol precursor in the fluid-transfer medium will be released into the channels from the activation surface 38, because the aerosol precursor is drawn into the one or more channels by way of the lower pressure. This effect is in addition to the effect of releasing the aerosol precursor from the activation surface 38 by way of heat conveyed from the heater. The drawing of the aerosol precursor from the activation surface 38 by way of the user sucking on the mouthpiece of the apparatus (or one the second end 18 of the aerosol carrier 14, if configured as a mouthpiece) may produce a dragging effect on the volumetric rate of flow experienced by the user during a suction action, i.e. the user may have to suck harder to achieve a same volumetric rate of flow. This effect may manifest itself as a similar physical sensation experienced by the user as those experienced from a traditional smoking or tobacco product.

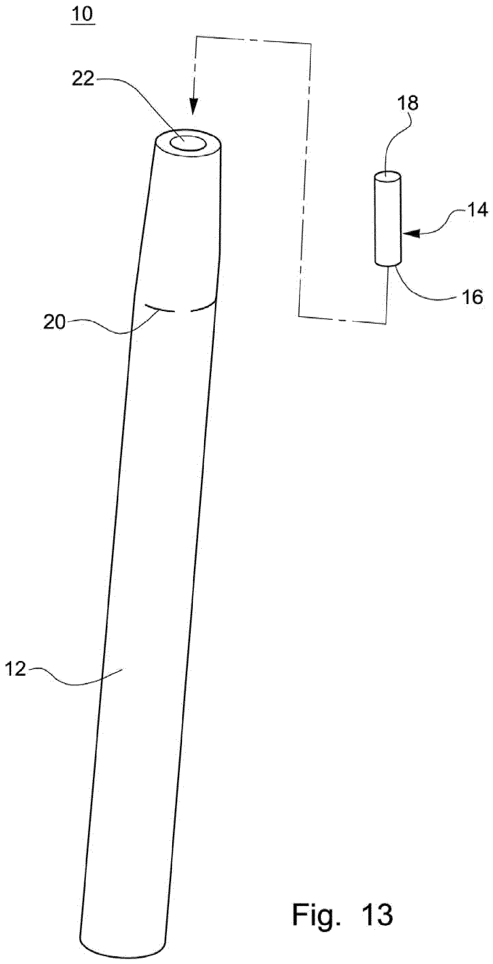

Figure 13 is an exploded perspective view illustration of a kit-of-parts for assembling an aerosol delivery system 10.

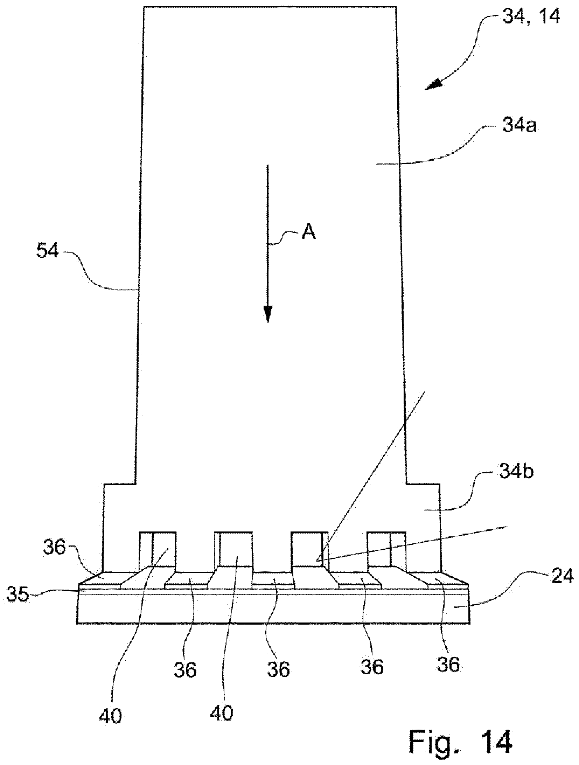

As will be appreciated, in the arrangements described above, the fluid-transfer article 34 is provided within a housing 32 of the aerosol carrier 14. In such arrangements, the housing of the carrier 14 serves to protect the aerosol precursor-containing fluid-transfer article 34, whilst also allowing the carrier 14 to be handled by a user without his/her fingers coming into contact with the aerosol precursor liquid retained therein. In such arrangements, it will be appreciated that the carrier 14 has a multi-part construction. In some cases this might be considered somewhat disadvantageous because it requires a relatively complicated assembly procedure which can be both time-consuming and expensive. Turning now to consider Figure 14, there is illustrated another possible aspect of the fluid-transfer article 34, which may be employed in some arrangements, and which may permit the creation of a significantly simplified carrier 14.

Figure 14 illustrates an alternative fluid-transfer article 34 in position adjacent a planar heater 24, such that the air flow channels 40 are positioned between the activation surface 38 and the heater 24. In the arrangement of Figure 14, the substrate forming the fluid-transfer article 34 again comprises a porous material where pores of the porous material hold, contain, carry, or bear the aerosol precursor material. It is envisaged, for example, that the same types of substrate material may be used in the arrangement illustrated in Figure 14 as in the previously-described arrangements. In particular, therefore, the porous material of the fluid-transfer article 34 may be a polymeric wicking material. However, in the arrangement illustrated in Figure 14, the substrate material includes an integrally formed peripheral wall 54.

It is proposed that the peripheral wall 54 may be formed by treating the outermost surface of the porous substrate material of the fluid-transfer article 34 so as to render the surface substantially liquid-impermeable. For example, it is envisaged that in some arrangements the substrate material may be locally heated so as to fuse the material and close up its internal pores in the localised region of the surface. Alternatively, it is envisaged that the substrate material may be treated by a sintering process in order to create the liquid-impermeable peripheral wall 54. The peripheral wall 54 may alternatively be created by a chemical treatment process to render the substrate material substantially liquid-impermeable in the region of its outermost surface. As will therefore be appreciated, the peripheral wall 54 may be considered to take the form of a skin formed from the material of the substrate itself.

The peripheral wall may be created in this manner so as to substantially completely circumscribe the substrate material. It is to be appreciated, however, that the activation surface 38 of the fluid-transfer article 34 will not be treated in this manner, thereby ensuring that it will retain the function described above in detail in cooperation with the heater 24. The thickness of the peripheral wall 54 formed from the substrate may vary depending on the desired physical properties of the fluid-transfer article 34. For example, a relatively thin wall 54 might be desirable in some circumstances, as this may retain some flexibility in the material, thereby providing a fluid-transfer article which will feel soft in the hands of a user. Alternatively, a relatively thick peripheral wall 54 might be desirable in arrangements where the wall 54 is required to provide some structural rigidity to the fluid-transfer article 34. The wall54 may therefore have a thickness of less than 3mm; or less than 2.5mm; or less than 2mm; or less than 1.5mm; or less than 1mm; or less than 0.9mm; or less than 0.8mm; or less than 0.7mm; or less than 0.6mm; or less than 0.5mm; or less than 0.4mm; or less than 0.3mm; or less than 0.2mm; or less than 0.1mm in some embodiments.

As will be appreciated, the liquid-impermeable nature of the resulting peripheral wall or skin means that the fluid-transfer article 34 may be handled by a user without getting his or her fingers wet from the aerosol precursor liquid retained therein. This opens up the possibility of the fluid-transfer article 34 being used without an enclosing housing 32, as was necessary in the previously-described arrangements. It is therefore envisaged that in some arrangements, the fluid-transfer article 34 may itself define an entire aerosol carrier 14. Furthermore, it is envisaged that in some embodiments, a fluid-transfer article 34 in accordance with this proposal may be provided in the form of a unitary monolithic element of substrate material and could, therefore, take the form of a single-piece consumable or carrier 14 for an aerosol-delivery system 10, which may be provided pre-filled with aerosol precursor liquid and which may be discarded when the initial volume of precursor has been used. A single-piece consumable of this type offers very significant advantages in terms of cost of manufacture, and from an environmental point of view.

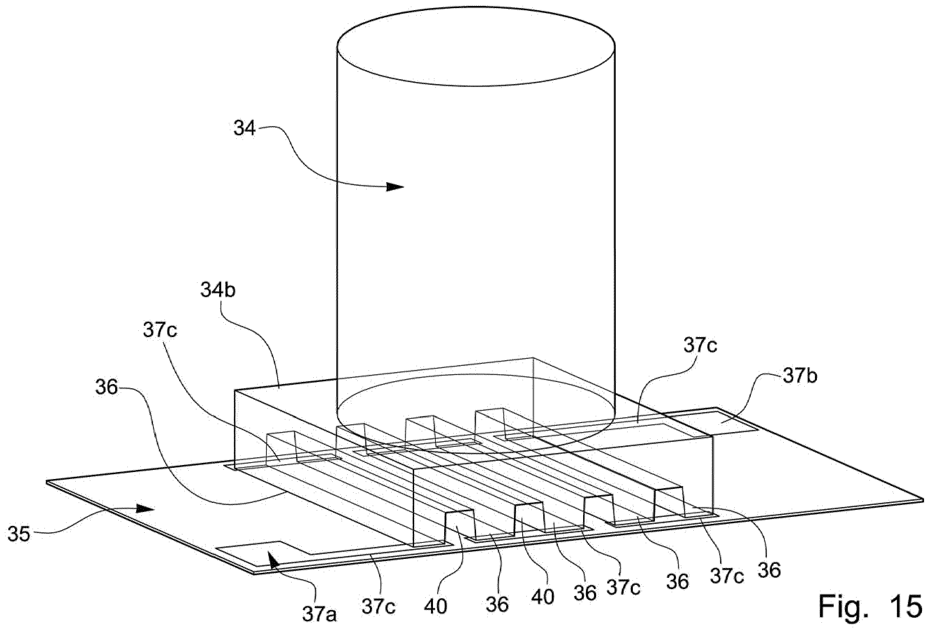

In order to illustrate the electrical connection of the heating elements 36, Figure 15 shows an arrangement corresponds to Figure 14, but in a perspective view, with part of the fluid-transfer article shown transparent (in reality, it will not be transparent). Figure 15 thus illustrates the channels 40 and the heating elements 36 which are aligned with the walls of the second region 34b on either side of the channels 40. Figure 15 also illustrates electrical contacts 37a and 37b on the substrate 35, which are connected to the heating elements 36 and which are connected to a source of electrical power for heating the heating elements 36. Conductive strips 37c then connect the terminals 37a and 37b with the heating elements 36, and connect the heating elements 36 to each other. The connection is such that the heating elements and conductive strips 37c form a zig-zag arrangement along the substrate 35. Other parts of the structure of Figure 15 correspond to those shown in Figure 14.

The porous layer may have a thickness of less than 5mm. In other embodiments it may have a thickness of: less than 3.5mm, less than 3mm, less than 2.5mm, less than 2mm, less than 1.9mm, less than 1.8mm, less than 1.7mm, less than 1.6mm, less than 1.5mm, less than 1.4mm, less than 1.3mm, less than 1.2mm, less than 1.1mm, less than 1 mm, less than 0.9mm, less than 0.8mm, less than 0.7mm, less than 0.6mm, less than 0.5mm, less than 0.4mm, less than 0.3mm, less than 0.2mm, or less than 0.1mm.

There has been described in the foregoing one or more proposals for an aerosol delivery system, and parts thereof, that avoids or at least ameliorates problems of the prior art.

In one or more optional arrangements, a fluid-transfer article 34 containing nicotine and/or nicotine compounds may be substituted or supplemented with a fluid-transfer article configured to provide a flavoured vapour and/or aerosol upon heating of the fluid-transfer article by the heater 24 of the apparatus 12. A precursor material for forming the flavoured vapour and/or aerosol upon heating is held within pores, spaces, channels and/or conduits within the fluid-transfer article. The precursor material may be extracted from a tobacco plant starting material using a supercritical fluid extraction process. Optionally, the precursor material is nicotine-free and comprises tobacco-flavours extracted from the tobacco plant starting material. Further optionally, the extracted nicotine-free precursor material (e.g. flavours only) could have nicotine added thereto prior to loading of the precursor material into the substrate of the carrier unit. Further optionally, flavours and physiologically active material may be extracted from plants other than tobacco plants.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof, but the invention is solely defined by the claims.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the scope of the invention as defined in the claims.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. An aerosol-generation apparatus (12) comprising a heater (24) and a fluid-transfer article (34), said fluid-transfer article (34) having a first region (34a) for holding an aerosol precursor and for transferring said aerosol precursor to an activation surface (38) of a second region (34b) of said article, said activation surface (38) being disposed and configured for thermal interaction with a heating surface of said heater (12); said second region (34b) comprising at least one discontinuity in said activation surface (38) to form a corresponding at least one channel (40) between said second region (34b) and said heating surface and being configured such that, when the fluid transfer article (34) is arranged with respect to said heating surface of the heater (24) for thermal interaction therebetween, the or each channel (40) opposes said heating surface, opens towards said heating surface, and provides an air-flow pathway across said heating surface; wherein the heater comprises a substrate (35) defining said heating surface, and at least one heating element (36) formed on a part of said heating surface, and **characterised in that** the or each channel (40) opposes a part of said heating surface other than said part of said heating surface on which the or each heating element is formed, such that the or each heating element is not aligned with any of the at least one channel, but is aligned with a part of the activation surface other than the at least one channel, so that the or each heating element is aligned with the part or parts of the activation surface which project towards the heater.

2. An aerosol-generation apparatus (12) according to claim 1, wherein said activation surface (38) is configured such that the or each said discontinuity is spaced apart from said heating surface.

3. An aerosol-generation apparatus (12) according to claim 1 or claim 2, wherein the or each said channel (40) is at least partly defined by a pair of spaced apart side walls, and an arcuate surface portion extending between said wall portions to form a ceiling portion of said channel (40).

4. An aerosol-generation apparatus (12) according to claim 3, wherein said arcuate surface portion blends smoothly with each of said side walls, thereby eliminating a sharp corner therebetween.

5. An aerosol-generation apparatus (12) according to claim 1 or claim 2, wherein the or each channel (40) is at least partially defined by a pair of spaced apart side walls and a flat surface portion, said flat surface portion extending between said wall portions to form a ceiling portion of said channel (40).

6. An aerosol-generation apparatus (12) according to claim 1 or claim 2, wherein the or each channel (40) is at least partially defined by a pair of side walls, said side walls being inclined relative to each other to meet at an apex portion of said channel (40).

7. An aerosol-generation apparatus (12) according to any one of claims 3 to 6, wherein said side walls are substantially planar.

8. An aerosol-generation apparatus (12) according to any preceding claim, wherein at least said second region (34b) is formed from a polymeric wicking material.

9. An aerosol-generation apparatus (12) according to claim 8, wherein said first and second regions (34a, 34b) are both formed from said polymeric wicking material.

10. An aerosol-generation apparatus (12) according to claim 8 or claim 9, wherein said polymeric wicking material is porous.

11. An aerosol-generation apparatus (12) according to any one of claims 8 to 10, wherein said polymeric wicking material is configured such that the pore diameter in said first region (34a) is greater than the pore diameter in said second region (34b).

12. An aerosol-generation apparatus (12) according to any one of claims 8 to 11, wherein said polymeric wicking material is heat resistant.

13. An aerosol-generation apparatus (12) according to any one of claims 8 to 12, wherein said polymeric wicking material is a hydrophilic material that is configured to transfer fluid from said first region (34a) to said second region (34b).

14. An aerosol-generation apparatus (12) according to any one of claims 8 to 13, wherein said polymeric wicking material is of greater hydrophilicity in said second region (34b) than said first region (34a).

15. An aerosol delivery system (10) comprising an aerosol-generation apparatus (12) according to any one of the preceding claims, and a carrier (14), the carrier (14) having a housing (26) containing said heater (24) and said fluid-transfer article (34).

16. An aerosol delivery system (10) according to claim 15, wherein said housing (26) has an inlet (50) and an outlet, said air-flow pathway extending to said inlet (50) and said outlet.

## Patentansprüche

1. Aerosolerzeugungsvorrichtung (12), umfassend ein Heizelement (24) und einen Fluidtransportartikel (34), wobei der Fluidtransportartikel (34) einen ersten Bereich (34a) zum Enthalten eines Aerosolvorläufers und zum Transport des Aerosolvorläufers an eine Aktivierungsoberfläche (38) eines zweiten Bereichs (34b) des Artikels umfasst, wobei die Aktivierungsoberfläche (38) zur thermischen Wechselwirkung mit einer Heizoberfläche des Heizelements (12) angeordnet und ausgelegt ist; wobei der zweite Bereich (34b) zumindest eine Unterbrechung in der Aktivierungsoberfläche (38) umfasst, um zumindest einen entsprechenden Kanal (40) zwischen dem zweiten Bereich (34b) und der Heizoberfläche auszubilden, und die so ausgelegt ist, dass, wenn der Fluidtransportartikel (34) in Bezug auf die Heizoberfläche des Heizelements (24) zur thermischen Wechselwirkung zwischen diesen angeordnet ist, der oder jeder Kanal (40) der Heizoberfläche gegenüberliegt, sich in Richtung der Heizoberfläche öffnet und einen Luftströmungspfad über die Heizoberfläche hinweg bereitstellt; wobei das Heizelement ein Substrat (35) umfasst, das die Heizoberfläche definiert, und wobei das zumindest eine Heizelement (36) auf einem Teil der Heizoberfläche ausgebildet ist, und **dadurch gekennzeichnet, dass** der oder jeder Kanal (40) einem Teil der Heizoberfläche gegenüberliegt, der nicht der Teil der Heizoberfläche ist, auf dem das oder jedes Heizelement ausgebildet ist, sodass das oder jedes Heizelement nicht mit einem beliebigen des zumindest einen Kanals fluchtend ausgerichtet ist, sondern mit einem Teil der Aktivierungsoberfläche fluchtend ausgerichtet ist, der nicht der zumindest eine Kanal ist, sodass das oder jedes Heizelement mit dem Teil oder den Teilen der Aktivierungsoberfläche fluchtend ausgerichtet ist, die in Richtung des Heizelements vorstehen.

2. Aerosolerzeugungsvorrichtung (12) nach Anspruch 1, wobei die Aktivierungsoberfläche (38) so ausgelegt ist, dass die oder jede Unterbrechung von der Heizoberfläche beabstandet ist.

3. Aerosolerzeugungsvorrichtung (12) nach Anspruch 1 oder Anspruch 2, wobei der oder jeder Kanal (40) zumindest teilweise durch ein Paar von beabstandeten Seitenwänden definiert ist, und wobei sich ein bogenförmiger Oberflächenabschnitt zwischen den Seitenwänden erstreckt, um einen Deckenabschnitt des Kanals (40) auszubilden.

4. Aerosolerzeugungsvorrichtung (12) nach Anspruch 3, wobei der bogenförmige Oberflächenabschnitt glatt in jede der Seitenwände übergeht, wodurch eine scharfe Ecke zwischen diesen vermieden wird.

5. Aerosolerzeugungsvorrichtung (12) nach Anspruch 1 oder Anspruch 2, wobei der oder jeder Kanal (40) zumindest teilweise durch ein Paar von beabstandeten Seitenwänden und einen flachen Oberflächenabschnitt definiert ist, wobei sich der flache Oberflächenabschnitt zwischen den Seitenwandabschnitten erstreckt, um einen Deckenabschnitt des Kanals (40) auszubilden.

6. Aerosolerzeugungsvorrichtung (12) nach Anspruch 1 oder Anspruch 2, wobei der oder jeder Kanal (40) zumindest teilweise durch ein Paar von Seitenwänden definiert ist, wobei die Seitenwände relativ zueinander geneigt sind, um sich an einem Scheitelpunktabschnitt des Kanals (40) zu treffen.

7. Aerosolerzeugungsvorrichtung (12) nach einem der Ansprüche 3 bis 6, wobei die Seitenwände im Wesentlichen planar sind.

8. Aerosolerzeugungsvorrichtung (12) nach einem der vorangegangenen Ansprüche, wobei zumindest der zweite Bereich (34b) aus einem polymeren Dochtmaterial ausgebildet ist.

9. Aerosolerzeugungsvorrichtung (12) nach Anspruch 8, wobei der erste und der zweite Bereich (34a, 34b) beide aus dem polymeren Dochtmaterial ausgebildet sind.

10. Aerosolerzeugungsvorrichtung (12) nach Anspruch 8 oder Anspruch 9, wobei das Polymerdochtmaterial porös ist.

11. Aerosolerzeugungsvorrichtung (12) nach einem der Ansprüche 8 bis 10, wobei das polymere Dochtmaterial so ausgelegt ist, dass der Porendurchmesser in dem ersten Bereich (34a) größer ist als der Porendurchmesser in dem zweiten Bereich (34b).

12. Aerosolerzeugungsvorrichtung (12) nach einem der Ansprüche 8 bis 11, wobei das polymere Dochtmaterial hitzebeständig ist.

13. Aerosolerzeugungsvorrichtung (12) nach einem der Ansprüche 8 bis 12, wobei das polymere Dochtmaterial ein hydrophiles Material ist, das ausgelegt ist, um Fluid aus dem ersten Bereich (34a) in den zweiten Bereich (34b) zu transportieren.

14. Aerosolerzeugungsvorrichtung (12) nach einem der Ansprüche 8 bis 13, wobei das polymere Dochtmaterial in dem zweiten Bereich (34b) eine größere Hydrophilie aufweist als in dem ersten Bereich (34a).

15. Aerosolzufuhrsystem (10), umfassend eine Aerosolerzeugungsvorrichtung (12) nach einem der vorangegangenen Ansprüche und einen Träger (14), wobei der Träger (14) ein Gehäuse (26) aufweist, das das Heizelement (24) und den Fluidtransportartikel (34) enthält.

16. Aerosolzufuhrsystem (10) nach Anspruch 15, wobei das Gehäuse (26) einen Einlass (50) und einen Auslass aufweist, wobei sich der Luftströmungsweg zum Einlass (50) und zum Auslass erstreckt.

## Revendications

1. Appareil de génération d'aérosol (12) comprenant un dispositif de chauffage (24) et un article de transfert de fluide (34), ledit article de transfert de fluide (34) présentant une première région (34a) pour contenir un précurseur d'aérosol et pour transférer ledit précurseur d'aérosol jusqu'à une surface d'activation (38) d'une seconde région (34b) dudit article, ladite surface d'activation (38) étant disposée et configurée pour une interaction thermique avec une surface de chauffage dudit dispositif de chauffage (12) ; ladite seconde région (34b) comprenant au moins une discontinuité dans ladite surface d'activation (38) pour former au moins un canal correspondant (40) entre ladite seconde région (34b) et ladite surface de chauffage, et étant configurée de telle sorte que lorsque l'article de transfert de fluide (34) est agencé par rapport à ladite surface de chauffage du dispositif de chauffage (24) pour une interaction thermique entre ceux-ci, le ou chaque canal (40) est opposé à ladite surface de chauffage, s'ouvre vers ladite surface de chauffage, et fournit un trajet d'écoulement d'air à travers ladite surface de chauffage ; dans lequel le dispositif de chauffage comprend un substrat (35) définissant ladite surface de chauffage, et au moins un élément de chauffage (36) formé sur une partie de ladite surface de chauffage, et **caractérisé en ce que** le ou chaque canal (40) est opposé à une partie de ladite surface de chauffage autre que ladite partie de ladite surface de chauffage sur laquelle le ou chaque élément de chauffage est formé, de telle sorte que le ou chaque élément de chauffage n'est pas aligné avec l'un quelconque du au moins un canal, mais est aligné avec une partie de la surface d'activation autre que le au moins un canal, de telle sorte que le ou chaque élément de chauffage est aligné avec la partie ou les parties de la surface d'activation qui font saillie vers le dispositif de chauffage.

2. Appareil de génération d'aérosol (12) selon la revendication 1, dans lequel ladite surface d'activation (38) est configurée de telle sorte que la ou chaque dite discontinuité est espacée de ladite surface de chauffage.

3. Appareil de génération d'aérosol (12) selon la revendication 1 ou la revendication 2, dans lequel le ou chaque dit canal (40) est au moins partiellement défini par une paire de parois latérales espacées, et une partie de surface arquée s'étendant entre lesdites parties de paroi pour former une partie de plafond dudit canal (40).

4. Appareil de génération d'aérosol (12) selon la revendication 3, dans lequel ladite partie de surface arquée se fond en douceur avec chacune desdites parois latérales, en éliminant ainsi un coin aigu entre celles-ci.

5. Appareil de génération d'aérosol (12) selon la revendication 1 ou la revendication 2, dans lequel le ou chaque canal (40) est au moins partiellement défini par une paire de parois latérales espacées et une partie de surface plate, ladite partie de surface plate s'étendant entre lesdites parties de paroi pour former une partie de plafond dudit canal (40).

6. Appareil de génération d'aérosol (12) selon la revendication 1 ou la revendication 2, dans lequel le ou chaque canal (40) est au moins partiellement défini par une paire de parois latérales, lesdites parois latérales étant inclinées l'une par rapport à l'autre pour se rencontrer au niveau d'une partie de sommet dudit canal (40).

7. Appareil de génération d'aérosol (12) selon l'une quelconque des revendications 3 à 6, dans lequel lesdites parois latérales sont sensiblement planes.

8. Appareil de génération d'aérosol (12) selon l'une quelconque des revendications précédentes, dans lequel au moins ladite seconde région (34b) est formée à partir d'un matériau à effet de mèche polymère.

9. Appareil de génération d'aérosol (12) selon la revendication 8, dans lequel lesdites première et seconde régions (34a, 34b) sont toutes deux formées à partir dudit matériau à effet de mèche polymère.

10. Appareil de génération d'aérosol (12) selon la revendication 8 ou la revendication 9, dans lequel ledit matériau à effet de mèche polymère est poreux.

11. Appareil de génération d'aérosol (12) selon la revendication 6, dans lequel ledit matériau à effet de mèche polymère est configuré de telle sorte que le diamètre de pore dans ladite première région (34a) est supérieur au diamètre de pore dans ladite seconde région (34b).

12. Appareil de génération d'aérosol (12) selon l'une quelconque des revendications 8 à 11, dans lequel ledit matériau à effet de mèche polymère est thermorésistant.

13. Appareil de génération d'aérosol (12) selon l'une quelconque des revendications 8 à 12, dans lequel ledit matériau à effet de mèche polymère est un matériau hydrophile qui est configuré pour transférer un fluide de ladite première région (34a) à ladite seconde région (34b).

14. Appareil de génération d'aérosol (12) selon l'une quelconque des revendications 8 à 13, dans lequel ledit matériau à effet de mèche polymère est d'une plus grande hydrophilie dans ladite seconde région (34b) que dans ladite première région (34a).

15. Système de distribution d'aérosol (10) comprenant un appareil de génération d'aérosol (12) selon l'une quelconque des revendications précédentes, et un support (14) le support (14) présentant un boîtier (26) contenant ledit dispositif de chauffage (24) et ledit article de transfert de fluide.

16. Système de distribution d'aérosol (10) selon la revendication 15, dans lequel ledit logement (26) présente une entrée (50) et une sortie, ledit trajet d'écoulement d'air s'étendant vers ladite entrée (50) et ladite sortie.
